(19)

Europäisches
Patentamt
European
Patent Office
Office européen
des brevets

(11) EP 2 713 160 A2

(12) EUROPEAN PATENT APPLICATION

(43) Date of publication:
02.04.2014 Bulletin 2014/14

(51) Int Cl.:
*G01N 33/20* (2006.01)

(21) Application number: 13186701.2

(22) Date of filing: 30.09.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 01.10.2012 JP 2012219101

(71) Applicant: Hitachi Ltd.
Tokyo 100-8280 (JP)

(72) Inventors:
• ISOBE, Nobuhiro
  Tokyo, 100-8280, (JP)
• YASHIRODAI, Kenji
  Tokyo, 100-8280, (JP)

(74) Representative: Calderbank, Thomas Roger et al
Mewburn Ellis LLP
33 Gutter Lane
London
EC2V 8AS (GB)

(54) **Method and system for evaluating creep damage of high temperature component**

(57) A method of evaluating creep damage of a high temperature component which assesses the degree of creep damage of a high temperature component for use under a high temperature environment. In the method, temporal change in damage parameter of the high temperature component under a uniaxial condition and temporal change in multiaxiality of the high temperature component are obtained, and the temporal change in damage parameter is corrected by the temporal change in multiaxiality to assess the degree of creep damage of the high temperature component.

FIG. 1

EP 2 713 160 A2

**Description**

The present invention relates to a structure material which may be used in a fast reactor, a fossil plant, or the like, and to a method and system for evaluating creep damage of a high temperature component, which may be used in a high temperature region equal to or higher than hundreds of degrees and damaged from creep.

Of components constituting a fast reactor or a fossil plant, heat resistant steel or heat resistant alloy is used in a region where temperature in operation is equal to or higher than hundreds of degrees. The materials continue to be subjected to a load at high temperature over a long period of time and thus undergo damage, such as creep, creep-fatigue, or embrittlement, and life is decided by the degree of damage. Since the degree of damage differs depending on temperature, stress, environment, or the like, components designed in the same manner are different in the degree of damage or life depending on service conditions.

For this reason, in regard to these high temperature components, the damage is evaluated during a service operation, and residual life or the like is estimated, thereby assessing continuous use, replacement, or the like of components or a plant. In these high temperature components, in particular, there are many cases where damage by creep is important, and various methods of evaluating damage by creep are suggested.

In these methods, the relationship between a life ratio and hardness, electrical resistivity, or a parameter relating to creep void, such as an A parameter or a void area fraction, is obtained as a damage growth curve in advance. Hardness or an A parameter measured in an actual component is compared with the damage growth curve, thereby evaluating the damage.

As the background art of this technical field, JP-A-2003-65978 is known. JP-A-2003-65978 describes "a relational curve of average life of positron annihilation and a life ratio of a material is created (omitted) to assess the degree of damage, the life ratio, or residual life."

JP-A-2004-333389 is also known. JP-A-2004-333389 describes "the quantity ratio of $M_6C$ carbide with respect to $M_7C_3$ carbide contained in carbide is obtained, (omitted) the progress of creep damage is evaluated."

JP-A-2006-258621 is also known. JP-A-2006-258621 describes "hardness of a component surface is estimated, the amount of strain of the component is estimated from the relationship between hardness and the amount of strain created in advance, and creep damage is obtained from comparison with a creep curve separately obtained."

JP-A-2008-249732 is also known. JP-A-2008-249732 describes "data relating to the time of a component in service and hardness at this time is constructed, the relationship between the time and hardness is approximated from the constructed data by a linear approximation, statistical analysis based on the probability theory is added to the approximation to estimate hardness, (omitted) and the degree of creep damage is estimated from the estimated hardness."

JP-A-2009-92478 is also known. JP-A-2009-92478 describes "a void fraction on the surface of heat resistant steel is calculated, multiaxiality of heat resistant steel is normalized, and the degree of creep damage of heat resistant steel of an inspection target is assessed on the basis of a graph representing the correlation between a life fraction of heat resistant steel and a void fraction normalized with multiaxiality created in advance from a value obtained by normalizing a void fraction on the surface of heat resistant steel to be inspected with multiaxiality."

JP-A-2010-164430 is also known. JP-A-2010-164430 describes "the correlation between the amount of creep strain of a test material and a crystal orientation distribution is obtained in advance, and a crystal orientation distribution of an inspection material is measured and applied to the correlation obtained in advance, thereby estimating the amount of creep strain of the inspection material."

As an example of the damage growth curve, "the relational curve of the average life of positron annihilation and the life ratio of the material" of JP-A-2003-65978, "the relationship between hardness and the amount of strain created in advance" of JP-A-2006-258621, "the correlation between the amount of creep strain of the test material and the crystal orientation distribution" of JP-A-2010-164430, or the like may be used. These are mainly obtained by an experiment, there are many cases where the experiment is performed using a standard round bar test, and stress to be loaded is generally in the uniaxial state. Meanwhile, in an actual component, there are few cases where a uniaxial stress state is reached, and in general, a multiaxial stress field, such as biaxial tension or triaxial tension, is reached depending on the shape of the component or material discontinuity of a welded zone or the like.

In the multiaxial stress field, since deformation is constrained, the progress of strain is delayed compared to the uniaxial state. Meanwhile, ductility when a component is ruptured is reduced. For this reason, there is a possibility that damage evaluation by comparison of data relating to strain or deformation obtained by a test in the uniaxial state and a measured value in the actual component in the multiaxial state has a significant error. It is known that the occurrence or growth of creep void is accelerated in the multiaxial state, and in evaluation by a void fraction described in JP-A-2009-92478, it is difficult to apply data collected by a uniaxial test to the actual component as it is.

When handling stress multiaxiality, for example, in JP-A-2009-92478, the effect of stress multiaxiality is taken into consideration toward a method of evaluating damage based on void fraction. In this method, multiaxiality in a region to be evaluated is obtained by structural analysis, and a value obtained by dividing the void fraction measured in the region by multiaxiality is defined as the degree of damage and compared with a damage growth curve as reference, thereby evaluating damage. That is, evaluation is made assuming that the degree of damage changes in proportion to multiaxiality.

However, there is a problem in that, in many cases, the relationship between multiaxiality and damage is not linear. Although a region in an actual component where stress multiaxiality increases is a region, such as a notched portion, in which stress concentration occurs, or a region, such as a heat affected zone of weldment, which is locally softened compared to the surroundings, since multiaxiality changes over time in these regions, there is also a problem in that the shape of the damage growth curve changes depending on load conditions or the like.

Since multiaxiality is obtained by structural analysis, a specimen in which multiaxiality is reproduced may be created, and a correction factor for a damage value, such as an A parameter, may be directly obtained by a creep test. Meanwhile, multiaxiality changes over time, or high temperature instrument is generally used over a few years to a few decades, and it is difficult to perform an appropriate test many times.

An object of the invention is to provide a method and system for evaluating creep damage of a high temperature component according to a stress state of a component, such as stress multiaxiality, for a component which is made of heat resistant steel or heat resistant alloy, is used at high temperature, and undergoes damage from creep, thereby improving precision of residual life evaluation or damage evaluation.

In order to solve the above-described problem, for example, a method described in the appended claims is introduced.

This application includes multiple means for solving the above-described problem, and as an example, there is provided a method of evaluating creep damage of a high temperature component which assesses the degree of creep damage of a high temperature component for use under a high temperature environment, in which temporal change in damage parameter of the high temperature component under a uniaxial condition and temporal change in multiaxiality of the high temperature component are obtained, and the temporal change in damage parameter is corrected by the temporal change in multiaxiality to assess the degree of creep damage of the high temperature component.

According to the invention, it is possible to evaluate damage of a high temperature component with high precision. For this reason, the degree of damage or residual life of a component of a power generating installation, such as a fast reactor, a boiler, or a turbine is predicted in advance, and avoidance of unplanned outage or replacement of parts or the like is optimized, thereby reducing economic loss.

The invention is not limited to inspection in service, and if multiaxiality of a region to be evaluated is obtained by inelastic analysis even in a design phase, creep strength or life for use in evaluation is corrected, making it possible to make a design for reduction in the amount of materials or improvement of environmental performance by shape optimization or reduction in weight.

In the drawings:

Fig. 1 is an example of a configuration diagram of a damage evaluation system;
Figs. 2A and 2B are diagrams showing the relationship between multiaxiality and a damage growth curve;
Figs. 3A and 3B are diagrams showing an example of an implementation to obtain a damage growth curve;
Fig. 4 is a model diagram showing the state of creep deformation and rupture under a multiaxial condition;
Fig. 5 is a diagram showing an example of evaluating creep strength of a notched material as a multiaxial condition;
Fig. 6 is a diagram showing an example of a damage growth curve under a condition that multiaxiality is constant;
Fig. 7 is a diagram illustrating a damage growth curve corrected taking into consideration multiaxiality;
Fig. 8 is a diagram illustrating a damage growth curve under a multiaxial condition;
Fig. 9 is a diagram illustrating creep strength evaluation by stress corrected with an exponential function of multiaxiality;
Fig. 10 is a sectional view of a welded zone;
Fig. 11 is a diagram showing a strain distribution obtained by creep analysis of a welded zone;
Figs. 12A to 12C are distribution diagrams of strain, stress, multiaxiality in a thickness direction of a HAZ obtained by creep analysis of a welded zone;
Figs. 13A and 13B are diagrams showing a damage growth curve of a HAZ subjected to correction taking into consideration multiaxiality;
Fig. 14 is a configuration diagram of a damage evaluation system based on analysis;
Figs. 15A and 15B are diagrams illustrating a damage evaluation result of a HAZ taking into consideration correction based on multiaxiality.

Hereinafter, an example of the invention will be described referring to the drawings.

In the invention, for a high temperature component which is applied to an actual installation, such as a fossil plant, and has an operation record, a damage parameter representing the degree of damage is obtained, and evaluation and assessment are performed. In this case, a criteria of assessment is defined from a damage growth curve of multiaxiality corrected on the basis of a damage growth curve under a uniaxial condition.

For this reason, in the following description, first, the relationship between multiaxiality and a damage growth curve will be clarified, and a specific correction method will be then described. Initially, multiaxiality will be described.

First, a triaxiality factor TF is given by Expression (1).

$$ TF = \frac{\sigma_1 + \sigma_2 + \sigma_3}{1/\sqrt{2} \cdot \left( (\sigma_1 - \sigma_2)^2 + (\sigma_2 - \sigma_3)^2 + (\sigma_3 - \sigma_1)^2 \right)^{0.5}} \qquad (1) $$

In Expression (1), $\sigma_1$, $\sigma_2$, and $\sigma_3$ are three components of principal stress in a high temperature component. For example, if Expression (1) is computed under $\sigma_1 \neq 0$ and $\sigma_2 = \sigma_3 = 0$ as the uniaxial condition in Expression (1), TF = 1. In Expression (1), as a biaxial tension condition and a triaxial tension condition, $\sigma_1$ and $\sigma_2 > 0$ and $\sigma_3 = 0$ for biaxial tension, and $\sigma_1$, $\sigma_2$, and $\sigma_3 > 0$ for triaxial tension are set, and in these cases, the triaxiality factor TF becomes equal to or greater than 1.

In a high temperature component of a fast reactor, a fossil plant, or the like, principal stress $\sigma_1$, $\sigma_2$, and $\sigma_3$ according to a load condition or a component temperature occurs. During actual operation over a long period of time, it is assumed that principal stress $\sigma_1$, $\sigma_2$, and $\sigma_3$ changes with gradual inelastic deformation by creep or the like or with change in temperature and temperature distribution, and accordingly, it is presumed that the triaxiality factor TF changes. The transition of principal stress over time can be preliminarily computed by structural analysis according to a fast reactor, a fossil plant, and an application place.

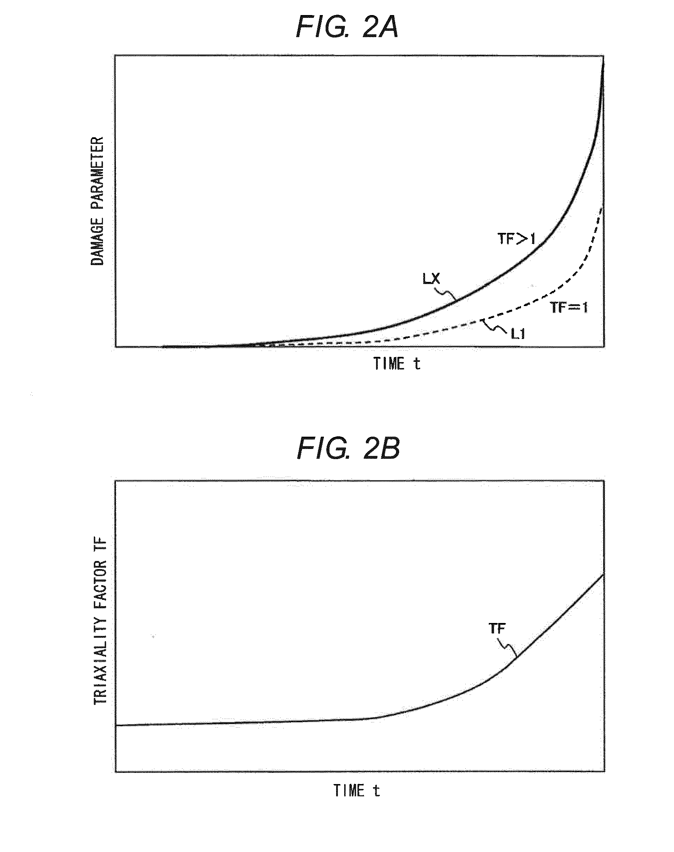

Figs. 2A and 2B are diagrams showing the relationship between a triaxiality factor TF and a damage growth curve L. Fig. 2B shows change in the triaxiality factor TF obtained by structural analysis over the time t. The triaxiality factor TF increases over the time to

In Fig. 2A, the horizontal axis represents a time and the vertical axis represents a damage parameter. The damage parameter increases over time, and a curve representing an increase tendency is a damage growth curve. A broken line L1 in Fig. 2A represents a damage growth curve when an operation continues in a uniaxial state (TF = 1).

The damage growth curve L1 in the uniaxial state (TF = 1) is obtained by a standard creep test on a smooth bar specimen as the relationship between a damage parameter, such as an A parameter, and time or a life fraction. In the drawing, the damage growth curve L1 in the uniaxial state (TF = 1) represents a tendency to increase over time.

For this reason, a high temperature component which starts to operate in the uniaxial state (TF = 1) from the beginning not only includes a factor of increase in a damage parameter over time, and increases twice by an increase in the triaxiality factor TF over time. In a damage growth curve LX in Fig. 2A, a factor over time of the triaxiality factor TF is added to the damage growth curve L1 which temporally changes intrinsically. The triaxiality factor TF of the damage growth curve LX is equal to or greater than 1.

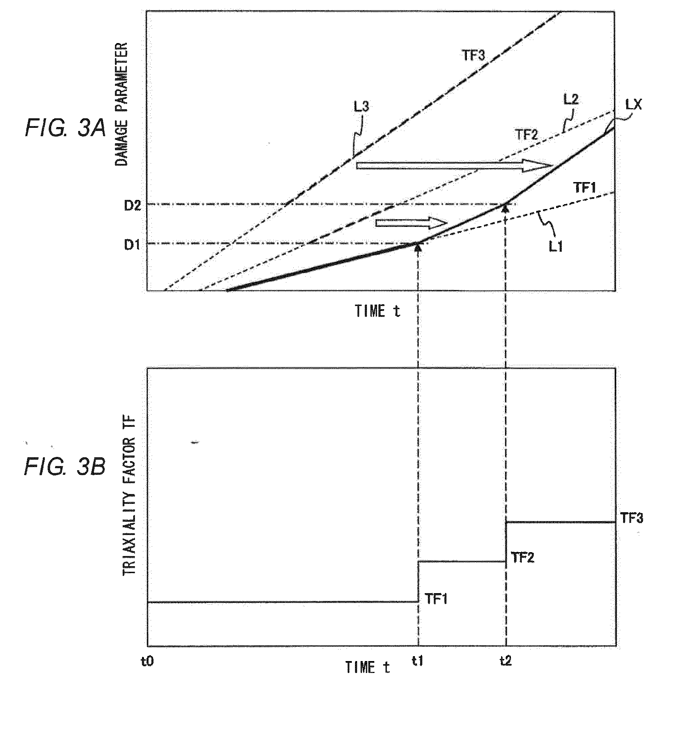

In the invention, a damage growth curve LX to which the influence of change in the triaxiality factor TF is added is estimated. Hereinafter, a method of simply simulating and estimating the damage growth curve LX will be described. Figs. 3A and 3B illustrate an example of an implementation to obtain the damage growth curve LX in Fig. 2A.

In the implementation of Figs. 3A and 3B, the relationship between a damage parameter and time is represented by a line. For example, in a graph of Fig. 3A, lines L1, L2, and L3 having different slopes are prepared. The lines L1, L2, and L3 have the triaxiality factor TF of 1, 2, and 3, and express the damage growth curve L when an operation continues in this state.

In this implementation, the triaxiality factor TF changes on a step with respect to time. That is, the curve of the gradually increasing triaxiality factor TF in Fig. 2B is expressed in Fig. 3B such that the triaxiality factor TF is 1 (referred to as TF1) from the time t0 to the time t1, the triaxiality factor TF is 2 (referred to as TF2) from the time t1 to the time t2, and the triaxiality factor TF is 3 (referred to as TF3) from the time t2 to the time t3.

In Fig. 3A, the lines L1, L2, and L3 represent the relationship between a damage parameter and time when the triaxiality factor TF is transited in a constant state of TF1, TF2, and TF3. The lines L1, L2, and L3 are expressed by lines whose slope is large as the triaxiality factor TF is large.

In the implementation of the damage growth curve LX, from the relationship between the assumed lines L1, L2, and L3 and the assumed triaxiality factors TF1, TF2, and TF3, it is assumed that an operation of a high temperature component is first done from the time t0 in a state where the triaxiality factor TF is TF1, damage progresses, and the multiaxiality changes from TF1 to TF2 at the time t1. In regard to a value of a damage parameter at this time, D1 is obtained as a value at the time t1 of the line L1 of Fig. 3A.

In the next step, it is assumed that the operation of the high temperature component in a state where the triaxiality factor is TF2 is done from the time t1. Although a damage parameter in this operation is represented by the line L2 of Fig. 3A, in this case, it is assumed that the damage parameter D1 at the time t1 is set as an initial value, and a subsequent damage parameter is defined with the slope of the line L2.

As the result of the operation of the high temperature component in a state where the triaxiality factor is TF2, the multiaxiality changes from TF2 to TF3 at the time t2. In regard to a damage parameter at this time, D2 is obtained as a value at the time t2 of the bold line LX of Fig. 3A.

In the next step, it is assumed that the operation of the high temperature component in a state where the triaxiality factor is TF3 is done from the time t2. Although a damage parameter in this operation is represented by the line L3 of Fig. 3A, in this case, it is assumed that the damage parameter D2 at the time t2 is set as an initial value, and a damage parameter is defined with the slope of the line L3.

In this way, finally, the bold line LX in Fig. 3A is obtained, and this line simulates the damage growth curve LX in Fig. 2A. When actually obtaining the damage growth curve LX by the simulation, it is necessary to define the time at which the triaxiality factor TF on the horizontal axis changes, and to find out the slopes of the lines L1, L2, and L3. The damage growth curves L1, L2, and L3 when multiaxiality is constant are obtained by the following way of thinking.

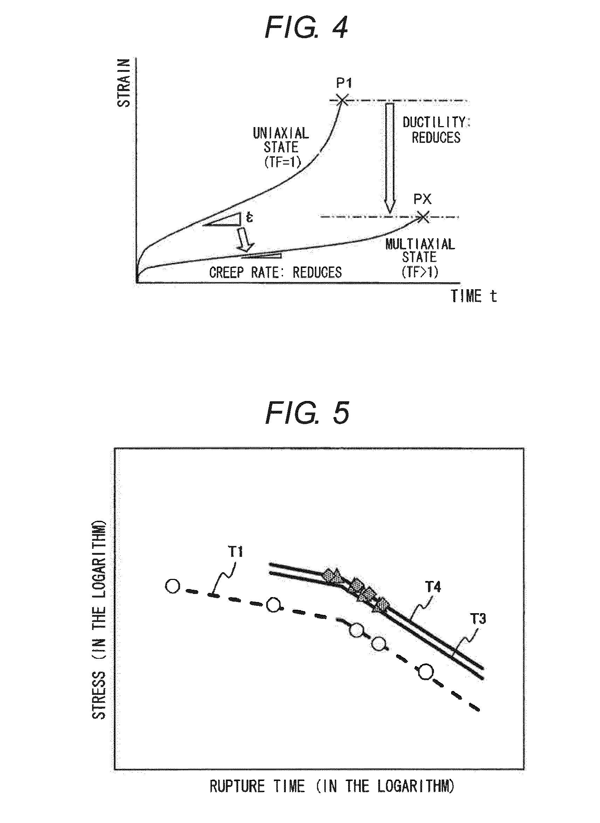

When the slopes of the lines L1, L2, and L3 are found out, the phenomenon of creep deformation and rupture is referenced. First, it is known that, under a multiaxial condition, creep rate and creep ductility are lowered compared to a uniaxial condition. Creep deformation and rupture under the multiaxial condition can be described with a model shown in Fig. 4.

In Fig. 4, the horizontal axis represents the time t, and the vertical axis represents strain. In this drawing, P1 represents a rupture point under a uniaxial condition, and PX represents a rupture point under a multiaxial condition. According to this characteristic, for example, while strain at the time of rupture under the uniaxial condition is large, the time leading to rupture is t10. In contrast, while the creep rate and creep ductility are lowered under the multiaxial condition, strain at the time of rupture is smaller than strain under the uniaxial condition, and the time leading to rupture is extended as indicated by t11.

That is, it is considered that, while the progress of the creep rate is delayed under the multiaxial condition compared to the uniaxial condition, ductility is lowered simultaneously, and life is defined with the balance between the creep rate and ductility. In order to numerically express the relationship that the creep rate decreases in a multiaxial stress field, equivalent stress $\sigma_{ec}$ of Expression (2) is considered.

$$\sigma_{ec} = \sigma_1 / TF^m \qquad (2)$$

Equivalent stress $\sigma_{ec}$ is represented by an expression in which maximum principal stress $\sigma_1$ is divided by a power of the triaxiality factor TF. In the multiaxial stress field, since the triaxiality factor TF > 1, if a coefficient m > 0, $\sigma_{ec}$ becomes smaller than $\sigma_1$. The fact that, even if $\sigma_1$ is the same, the creep rate is lowered under the condition that the triaxial coefficient m is high or creep strength increases can be expressed by Expression (2).

It is assumed that the following Monkman-Grant relationship is established between the creep rate $d\varepsilon_c/dt$ and the rupture time $t_r$.

$$\frac{d\varepsilon_c}{dt} \cdot t_r = C \qquad (3)$$

Here, C is a constant depending on a material or temperature, and since the left side is a product of a strain rate and time, can be considered as a constant relevant to ductility. As shown in Fig. 4, since ductility is also lowered under the multiaxial condition, as in Expression (2), the constant C can be expressed by Expression (4) using a relational expression of a reciprocal of the power of the triaxiality factor TF.

$$C = C_0 / TF^{m'} \qquad (4)$$

In Expression (3), it is assumed that the relationship between stress $\sigma_{ec}$ and the creep rate $d\varepsilon_c/dt$ follows the Norton's law.

$$\frac{d\varepsilon_c}{dt} = B \cdot \left(\sigma_{cc}\right)^n \qquad (5)$$

From the above, when stress (maximum principal stress) under the uniaxial condition is $\sigma_1$, stress (referred to as $\sigma_{1\_m}$) under the multiaxial condition at the same rupture time tr, Expression (6) is obtained.

$$\sigma_{1\_m} = \sigma_1 \cdot TF^{\frac{mn-m'}{n}} = \sigma_1 \cdot TF^{\alpha} \qquad (6)$$

That is, in this model, creep strength under the multiaxial condition becomes $TF^{\alpha}$ times greater than under the uniaxial condition. In the invention, the slopes of the lines L1, L2, and L3 are found out using the power $\alpha$ defined in the above-described manner.

An example where creep strength of a notched material under a multiaxial condition is assessed with the above assessment method is shown in Fig. 5. In Fig. 5, the horizontal axis represents the rupture time tr in the logarithm, and the vertical axis represents stress in the logarithm. Here, as a creep test result of low-alloy steel, strength (T3, T4) of a notched material whose shape is defined such that the triaxiality factor TF becomes 3 and 4 and strength T1 of a smoothing material (TF = 1) are displayed and compared.

The relationship between stress and rupture time can be substantially approximated to two lines while being divided into a shorter life region and a longer life region on a logarithmic graph. It can be understood that strength (T3, T4) under the condition that TF = 3 and TF = 4 becomes higher than strength T1 of the smoothing material (TF = 1). An increase in strength is not linear to the triaxiality factor TF. For this reason, if it is assumed that strength is simply in proportion to multiaxiality, this becomes a factor of error under the condition that multiaxiality is large. In the invention, solid lines (T3, T4) in the drawing are obtained by predicting strength of the notched material by Expression (6) from an experimental result of the uniaxial condition T1 indicated by a broken line with TF = 3 and TF = 4. In this case, in Expression (6), if $\alpha = 1/4.5$, prediction close to the experimental result is possible.

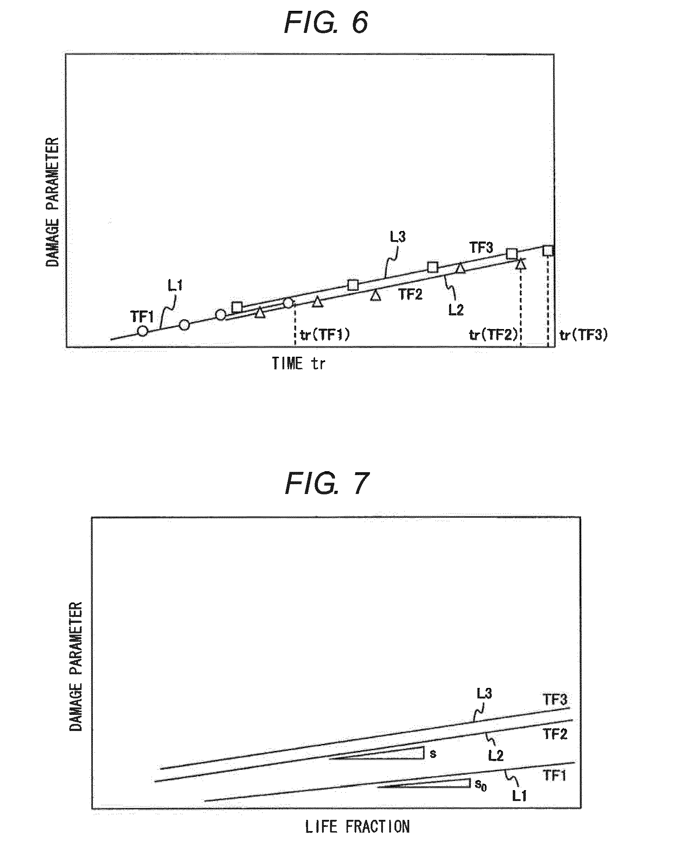

Next, an example of a damage growth curve under the condition that the triaxiality factor TF is constant is shown in Fig. 6. In Fig. 6, the horizontal axis represents the rupture time tr, and the vertical axis represents a damage parameter. Fig. 6 shows a case where the relationship between a damage parameter and time is expressed substantially by lines, and the slopes of these lines are not so changed depending on multiaxiality. The damage parameter on the vertical axis of Fig. 6 is based on a creep void. Accordingly, in this case, the occurrence and growth of the void are determined only by maximum principal stress. tr (TF1), tr (TF2), and tr (TF3) on the vertical axis of Fig. 6 are the rupture time tr in the respective axial conditions, and as will be expected from Expression (6), under the multiaxial condition, strength increases, that is, the rupture time tr when maximum principal stress is the same is extended.

For this reason, on a life fraction based on the rupture time tr, the damage parameter at the same time becomes larger under the multiaxial condition. Accordingly, when the horizontal axis is a life fraction, as shown in Fig. 7, the slope of the damage growth curve L changes by change in rupture time with the triaxiality factor TF.

From Fig. 5 described above, since the relationship between stress and rupture time is substantially linear on a logarithmic graph, the relationship between stress and rupture time can be approximated by an expression of a power. From this and Expression (6), the influence of multiaxiality toward rupture life can be expressed by an expression of a power. Accordingly, as shown in Fig. 7, if the slope of the damage growth curve in the uniaxial state (TF = 1) when the horizontal axis is a life fraction is $S_0$, the slope s at the time of the triaxiality factor TF can be predicted by the following expression.

$$s = s_0 \times TF^{\alpha'} \qquad (7)$$

Here, an exponent $\alpha'$ is obtained by taking into consideration an exponent when the relationship between rupture time and stress shown in Fig. 5 is approximated by a power over the exponent $\alpha$ of Expression (6). If this relationship is used, and if there are the relationship between creep rupture time and life and a few pieces of data having different triaxiality factors TF, a damage growth curve under a condition of an arbitrary triaxiality factor TF can be drawn.

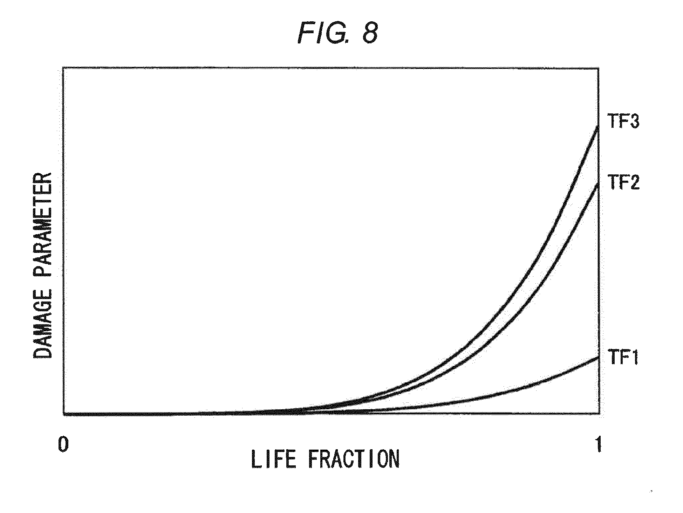

As another example, as shown in Fig. 8, a case where a damage growth curve can be displayed by a power of time like Expression (8).

$$D = K\left(t/t_r\right)^{\lambda} \qquad (8)$$

Here, D is a value of a damage parameter, $t_r$ is a rupture time, and K or $\lambda$ is obtained by fitting a function form of Expression (8) to experimental data.

In computing this expression, while the exponent $\lambda$ may also change depending on multiaxiality, the influence of the triaxiality factor TF toward the exponent $\lambda$ is taken into consideration, assessment becomes complicated. For this reason, even if precision of fitting is degraded to some extent, it is preferable to handle the exponent $\lambda$ as a value without depending on multiaxiality. In this case, only the coefficient K changes depending on the triaxiality factor TF, and the relationship between K and multiaxiality is formulated, thereby obtaining a damage growth curve in the multiaxial state.

In Expression (8), while a damage parameter is a function of a life fraction $t/t_r$, the same applies if an approximation is created as a function of the time t, and then correction is performed taking into consideration $t_r$ using Expression (6).

In the above example, while the influence of multiaxiality toward the creep rate or ductility of Expression (2) or (4) is represented by a power of multiaxiality, an exponential function may be used. In this case, for example, Expression (2) becomes Expression (9).

$$\sigma_{ec} = \sigma_1 \exp\left[A\left(1 - 1/TF\right)\right] \qquad (9)$$

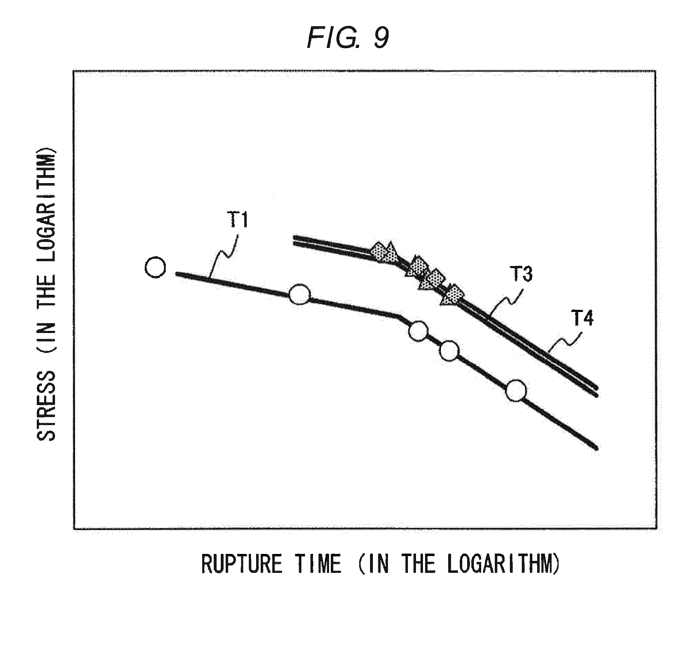

The form of Expression (9) is determined such that $\sigma_{ec} = \sigma_1$ with TF = 1. The same applies to other expressions. An example where data of Fig. 5 is fitted by Expression (9) is shown in Fig. 9. Similarly to Expression (2), it is understood that test data having different multiaxiality can be fitted.

A way of thinking which obtains an assessment value as a criteria of assessment for a damage parameter of a high temperature component having an operation record from a damage growth curve of multiaxiality obtained by correcting a damage growth curve under a uniaxial condition on the basis of multiaxiality has been described.

Next, a damage evaluation system which executes assessment and evaluation of a damage parameter of a high temperature component by, for example, a computer using the way of thinking will be described.

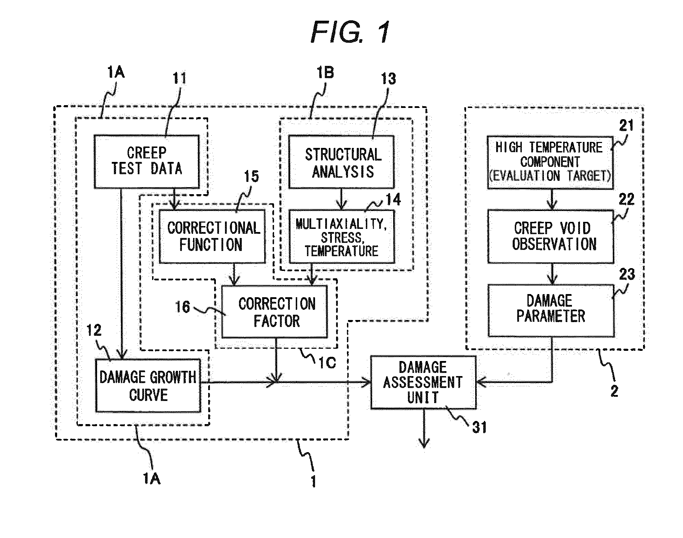

Fig. 1 is an example of a configuration diagram of a damage evaluation system according to the invention. This system includes a damage growth curve correction unit 1 which calculates multiaxiality and corrects a damage growth curve on the basis of multiaxiality, an actual parameter derivation unit 2 which performs actual inspection of a target component to obtain a damage parameter, such as an A parameter or a void area fraction, and a damage assessment unit 31 which assesses the degree of damage from the corrected damage growth curve and the actual damage parameter.

Of these, the actual parameter derivation unit 2 is not limited to the invention, and is the same as one for use in residual life assessment of a fossil plant or the like. As the way of thinking, for example, creep void observation 22 is executed on a high temperature component 21 to be assessed, and a damage parameter 23 is determined using the result. Although this method is well known and thus detailed description thereof will be omitted, in summary, a damage parameter which represents the degree of damage of a high temperature component to be actually operated in a plant or the like is obtained.

The damage growth curve correction unit 1 gives a criteria of evaluation for evaluating and assessing an actual damage parameter as a corrected damage growth curve. Since the damage evaluation system of the invention has a feature in that a damage growth curve is corrected on the basis of multiaxiality, hereinafter, description will be provided focusing on the damage growth curve correction unit 1. The damage growth curve correction unit 1 includes a damage growth curve derivation unit 1A which obtains, from experimental data or the like, a damage growth curve when multiaxiality is 1, a multiaxiality derivation unit 1B which obtains multiaxiality of the component, and a correction factor derivation unit 1C which calculates a correction factor for the damage growth curve when multiaxiality is 1 on the basis of the obtained multiaxiality.

Of these, in regard to the damage growth curve derivation unit 1A, a technique disclosed in JP-A-2003-65978, JP-A-2006-258621, JP-A-2010-164430, or the like may be used. For example, a damage growth curve 12 may be obtained using result data 11 of a creep test by, for example, a standard round bar test. The obtained damage growth curve is a curve when the triaxiality factor TF is 1. Actually, a slope $S_0$ of a damage growth curve L1 of Fig. 7 is output from the damage growth curve derivation unit 1A.

Next, the multiaxiality derivation unit 1B which obtains the triaxiality factor TF of the component will be described. In the multiaxiality derivation unit 1B of Fig. 1, computation 14 of multiaxiality from stress in the high temperature component obtained by structural analysis 13 is executed. Actually, for example, the multiaxiality derivation unit 1B executes Expression (1) to obtain the triaxiality factor TF. Since the triaxiality factor TF changes depending on the shape or material of a target component, it is necessary to obtain the triaxiality factor TF by inelastic analysis taking into consideration creep or elastic deformation, and processing for obtaining the triaxiality factor TF is executed by the multiaxiality derivation unit 1B.

The correction factor derivation unit 1C determines a specific amount of correction when correcting the uniaxial damage growth curve L1 according to multiaxiality. A correctional function derivation unit 15 of the correction factor derivation unit 1C obtains the exponent $\alpha'$ of Expression (7) using creep test data 11. A correction factor derivation unit 16 executes Expression (7) to obtain the slope S at the time of the triaxiality factor TF.

The damage assessment unit 31 computes the damage growth curve LX from the slope $S_0$ of the damage growth curve L1 and the slope S at the time of the triaxiality factor TF by applying the method described referring to Figs. 3A and 3B or the like, defines a damage parameter determined by the damage growth curve LX under the multiaxial condition as a criteria of assessment, performs comparison with the damage parameter of the actual high temperature component obtained from the actual parameter derivation unit 2, and outputs an assessment result. There are various methods which compute the damage growth curve LX, and the way of thinking of Figs. 3A and 3B is not necessarily used.

As an example of the evaluation method of the invention, an application example to creep damage evaluation of a welded joint of ferrite heat resistant steel to be used as a high temperature structure material of a fossil boiler or a fast reactor will be hereinafter described.

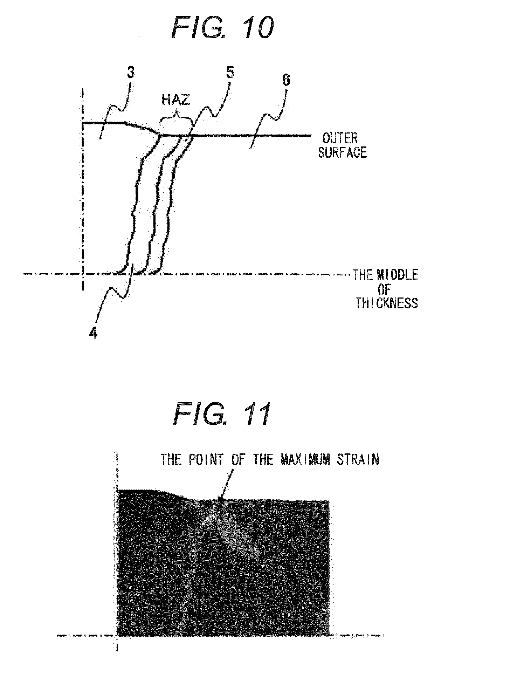

Fig. 10 is a sectional view of a welded joint. In Fig. 10, the upper side is an outer surface, the lower side is the middle of the thickness, and a portion where a base metal 6 is welded with a weld metal 3 is shown. In this case, while there are heat affected zones (HAZ) 4 and 5 between the base metal 6 and the weld metal 3, in the ferrite heat resistant steel, it is known that the region 5 of the HAZ on the base metal 6 side is softened compared to the surroundings and strain is likely to be concentrated. The region 5 is called a fine grain HAZ because a grain size is small. The region 4 of the HAZ on the weld metal 3 side is called a coarse grain HAZ 4 since a grain size is relatively large.

As an example of a result of creep analysis when the fine grain HAZ 5 is given a characteristic softer than the base metal 6 and the weld metal 3, the distribution of creep strain is shown in Fig. 11. It is understood that the maximum value of creep strain is generated in a region close to the outer surface of the fine grain HAZ 5, and strain is concentrated on the coarse grain HAZ 5.

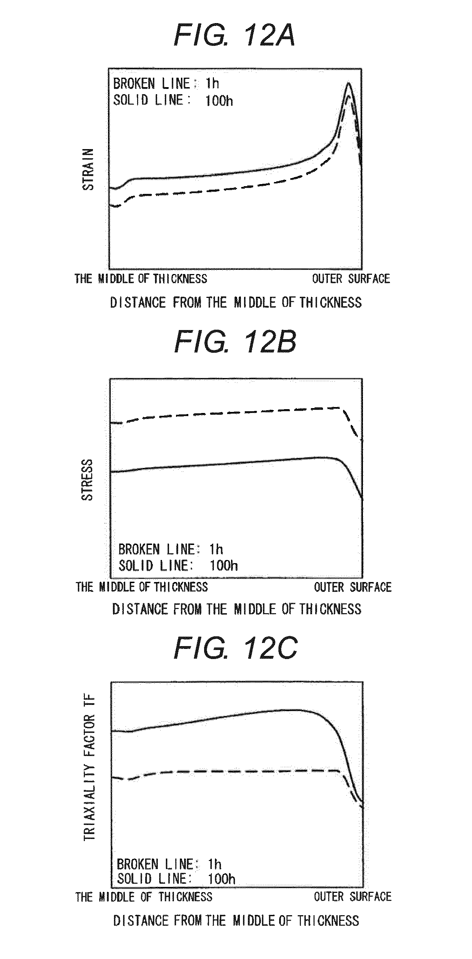

The distribution in the thickness direction of strain, stress, and multiaxiality in the fine grain HAZ 5 is shown in Figs. 12A to 12C. In Figs. 12A to 12C, the horizontal axis represents the thickness direction, the right side represents an outer surface, and the left side represents the middle of the thickness. In these graphs, a broken line and a solid line respectively represent the distribution of strain, stress, and multiaxiality for one hour from the start of creep and after 100 hours have elapsed.

When synthetically examining these amounts, the following can be confirmed. First, while strain increases intensively near the outer surface, stress and multiaxiality increase to the whole in the thickness. The position where multiaxiality is maximal changes slightly over time, and is substantially aligned with a position where stress is maximal.

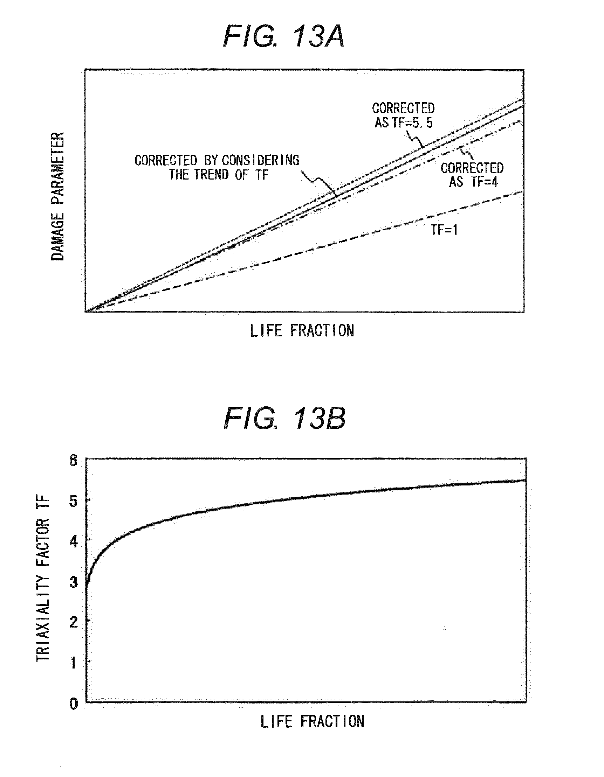

Accordingly, correction of a damage growth curve is performed using multiaxiality at a position where stress is maximal. An example which the correction is performed is shown in Figs. 13A and 13B. Fig. 13A represents the relationship between a life fraction and a damage parameter, and Fig. 13B represents the relationship between a life fraction and multiaxiality.

The relationship between a damage parameter and time under the condition that the triaxiality factor TF is constant is given in Fig. 6. Fig. 13B shows temporal change in the triaxiality factor TF obtained by analysis, in which the triaxiality factor TF is about 3 immediately after the start of creep, then gradually increases, and is transited to about 5.5 since the medium term of life. Accordingly, a solid line in the drawing is obtained by correcting the damage growth curve of TF = 1 indicated by a broken line in Fig. 13A. A one-dot-chain line and a dotted line show the result of correction by TF = 4 and TF = 5.5. With this, it is understood that, when TF is equal to or greater than 4, there is no significant difference even if TF is constant. It can be said that, when TF varies in a range of 1 to 3, variation in damage growth curve by multiaxiality increases.

A damage growth curve obtained by the above procedure is compared with a damage parameter, such as an A parameter or a void area fraction, obtained by inspection of a target component, making it possible to perform damage evaluation in conformity with the stress state of the component.

Although in the foregoing example, a damage evaluation method using an inspection result of a target component has been described, creep strength evaluation taking into consideration multiaxiality can be applied to damage evaluation based on analysis during design or the like.

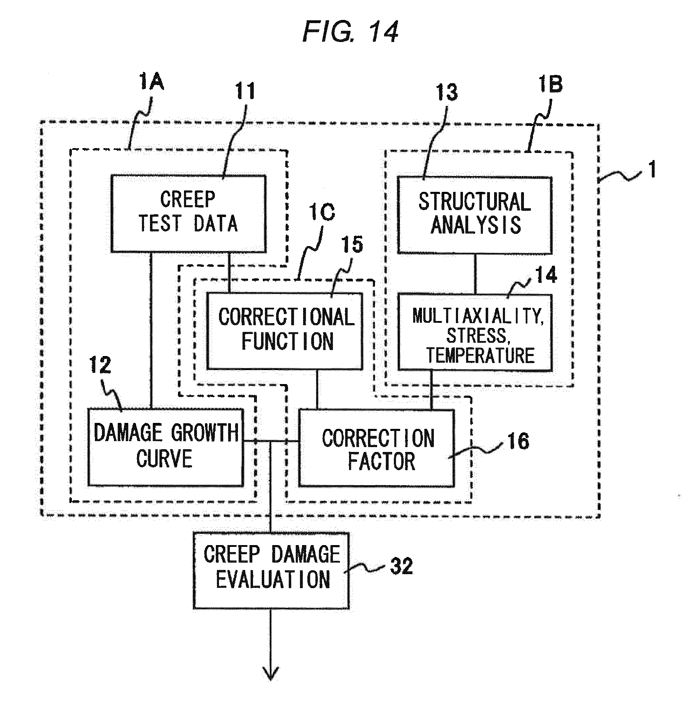

The configuration of an analytic damage evaluation system is shown in Fig. 14. A damage growth curve in an evaluation system 1 taking into consideration correction by multiaxiality in the damage evaluation system of Fig. 1 is substituted with a creep rupture time curve. In the system configuration of Fig. 14, the damage assessment unit 31 is substituted with a creep damage evaluation unit 32. In this case, a creep rupture time curve shows the relationship between stress and the creep rupture time under the uniaxial condition indicated by the broken line in Fig. 5.

In analytic damage evaluation, creep damage $D_c$ is evaluated by the following expression using temporal change in stress obtained by analysis.

$$D_c = \int dt/t_r(\sigma, T) \qquad (10)$$

On the right side, when temperature and stress for a minute time increment dt are $\sigma$ and T, creep damage accumulated for the time increment dt is obtained by the ratio of dt and the rupture time $t_r$ with stress $\sigma$ and temperature T, that is, $dt/t_r$, and integrated in an assumed period to obtain the creep damage $D_c$. The rupture time is obtained using stress and the creep rupture time curve, and stress to be used is corrected on the basis of the relationship of Expression (6).

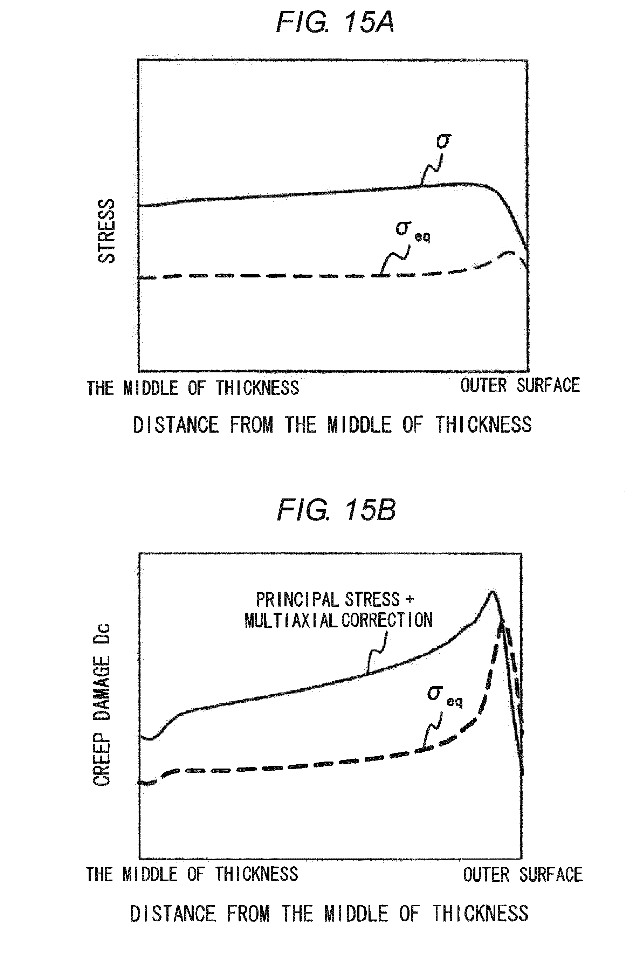

An example where creep damage of the welded joint shown in Fig. 10 is evaluated using a stress analysis result is shown in Figs. 25A and 15B. In Figs. 15A and 15B, the horizontal axis represents the position in the thickness direction, and the vertical axis represents stress and the creep damage Dc. In analytic creep damage evaluation, there are many cases where von Mises equivalent stress $\sigma_{eq}$ is used as stress for use in evaluation.

$$\sigma_{eq} = \frac{1}{\sqrt{2}}\sqrt{(\sigma_1 - \sigma_2)^2 + (\sigma_2 - \sigma_3)^2 + (\sigma_3 - \sigma_1)^2} \qquad (11)$$

Fig. 15A shows comparison between the equivalent stress $\sigma_{eq}$ and the distribution in the thickness of the principal stress $\sigma$, and it is understood that the value of the equivalent stress $\sigma_{eq}$ is smaller than the principal stress $\sigma$ and the degree of concentration near the surface increases. In evaluation by the equivalent stress $\sigma_{eq}$, in general, multiaxiality is not taken into consideration.

The result of evaluation of creep damage with the equivalent stress $\sigma_{eq}$ and the principal stress $\sigma$ is shown in Fig. 15B. Figs. 15A and 15B show the distribution in the thickness. While multiaxiality is not taken into consideration in evaluation by the equivalent stress $\sigma_{eq}$, in the case of principal stress, stress is corrected taking into consideration of multiaxiality shown in Figs. 12A to 12C, and evaluation is performed. While the maximum value of creep damage is generated inside a little from the outer surface with equivalent stress and principal stress, damage by principal stress increases in the thickness.

In the welded joint of ferrite heat resistant steel, while it is reported that the progress of creep damage in the thickness is fast, and damage by the fast progress of creep damage is problematic, in evaluation by equivalent stress, it is understood that damage is concentrated near the outer surface and does not correspond to a damage state reported in an actual component. If a damage value taking into consideration the effect of multiaxiality toward principal stress has a large value even in the thickness compared to equivalent stress.

As described above, correction of stress taking into consideration multiaxiality is performed, making it possible to achieve high precision of damage evaluation according to various stress states.

**Claims**

1. A method of evaluating creep damage of a high temperature component which assesses the degree of creep damage of a high temperature component for use under a high temperature environment,
   wherein temporal change in damage parameter of the high temperature component under a uniaxial condition and temporal change in multiaxiality of the high temperature component are obtained, and the temporal change in damage parameter is corrected by the temporal change in multiaxiality to assess the degree of creep damage of the high temperature component.

2. The method according to claim 1,

wherein the temporal change in damage parameter of the high temperature component under the uniaxial condition is obtained by a creep test, and the temporal change in multiaxiality of the high temperature component is obtained by structural analysis of the high temperature component.

3. The method according to claim 1,
wherein a correction factor when correcting the temporal change in damage parameter by the temporal change in multiaxiality is defined by a power of the multiaxiality.

4. The method according to claim 1,
wherein a correction factor when correcting the temporal change in damage parameter by the temporal change in multiaxiality is defined by an exponential function of the multiaxiality.

5. The method according to claim 1,
wherein the temporal change in damage parameter of the high temperature component is obtained as a damage growth curve of the damage parameter.

6. The method according to claim 1,
wherein the method of evaluating creep damage of the high temperature component is a method of evaluating creep damage which analytically obtains creep damage on the basis of stress or a temporal history of strain obtained by structural analysis, and performs correction by the temporal change in multiaxiality.

7. A method of evaluating creep damage of a high temperature component which is made of heat resistant steel or heat resistant alloy and used at high temperature over a long period of time,
wherein an influence of stress multiaxiality with respect to a growth rate of a parameter for use in assessing the degree of damage is formulated experimentally in advance, a growth rate of creep damage is corrected using the relationship between multiaxiality obtained by structural analysis of the high temperature component and time, and the degree of creep damage of the component is assessed from the relationship between creep damage obtained by correction and time.

8. A system for evaluating creep damage of a high temperature component which assesses the degree of creep damage of the high temperature component for use under a high temperature environment, the system comprising:

a damage growth curve correction unit including a damage growth curve derivation unit which obtains, from experimental data, a damage growth curve when multiaxiality is 1, a multiaxiality derivation unit which obtains multiaxiality of the high temperature component, and a correction factor derivation unit which calculates a correction factor with respect to the damage growth curve when multiaxiality is 1 on the basis of the obtained multiaxiality;
an actual parameter derivation unit which obtains a damage parameter by actual inspection of the high temperature component; and
a damage assessment unit which performs damage assessment from a corrected damage growth curve and an actual damage parameter.

EP 2 713 160 A2

*FIG. 1*

## FIG. 2A

## FIG. 2B

FIG. 3A

FIG. 3B

## FIG. 4

## FIG. 5

## FIG. 6

## FIG. 7

FIG. 8

## FIG. 9

## FIG. 10

3   5   6

HAZ

OUTER
SURFACE

THE MIDDLE
OF
THICKNESS

4

## FIG. 11

THE POINT OF THE MAXIMUM STRAIN

## FIG. 12A

BROKEN LINE: 1h
SOLID LINE: 100h

STRAIN

THE MIDDLE OF THICKNESS    OUTER SURFACE

DISTANCE FROM THE MIDDLE OF THICKNESS

## FIG. 12B

STRESS

BROKEN LINE: 1h
SOLID LINE: 100h

THE MIDDLE OF THICKNESS    OUTER SURFACE

DISTANCE FROM THE MIDDLE OF THICKNESS

## FIG. 12C

TRIAXIALITY FACTOR TF

BROKEN LINE: 1h
SOLID LINE: 100h

THE MIDDLE OF THICKNESS    OUTER SURFACE

DISTANCE FROM THE MIDDLE OF THICKNESS

## FIG. 13A

## FIG. 13B

## FIG. 14

## FIG. 15A

STRESS

$\sigma$

$\sigma_{eq}$

THE MIDDLE OF THICKNESS          OUTER SURFACE

DISTANCE FROM THE MIDDLE OF THICKNESS

## FIG. 15B

CREEP DAMAGE Dc

PRINCIPAL STRESS +
MULTIAXIAL CORRECTION

$\sigma_{eq}$

THE MIDDLE OF THICKNESS          OUTER SURFACE

DISTANCE FROM THE MIDDLE OF THICKNESS

**EP 2 713 160 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003065978 A
- JP 2004333389 A
- JP 2006258621 A
- JP 2008249732 A
- JP 2009092478 A
- JP 2010164430 A